# EUROPEAN PATENT APPLICATION

(11) **EP 4 803 022 A1**
(43) Date of publication of application: **09.09.2026**
(21) Application number: 25161193.5
(22) Date of filing: 03.03.2025
(51) Int. Cl.: A61B 34/00, A61B 34/37

(54) **SYSTEM AND METHOD FOR CONTROLLING A PLURALITY OF COAXIAL ENDOVASCULAR DEVICES**

(71) Applicant: Koninklijke Philips N.V., 5656 AE Eindhoven (NL)
(72) Inventor: TAN, Wee Kar, Eindhoven (NL); BALICKI, Marcin Arkadiusz, Eindhoven (NL); KYNE, Sean, Eindhoven (NL); KEENAN, Philip Joseph Jr., Eindhoven (NL); LANG, Sydney, Eindhoven (NL); VAN APELDOORN, Max, Eindhoven (NL)
(74) Representative: Philips Intellectual Property & Standards

(57) **Abstract**

The present invention relates to a system and method for controlling a plurality of coaxial endovascular devices. The system comprises a joystick with a plurality of buttons configured to be actuated while the joystick is being handled, and a controller configured to associate the plurality of buttons with the plurality of devices and to select and/or deselect one or more of the devices in accordance with the actuation of the buttons for controlling the one or more devices.

## Description

### FIELD OF THE INVENTION

The present invention relates to a system and method for controlling a plurality of coaxial endovascular devices.

### BACKGROUND OF THE INVENTION

In a robotics endovascular procedure, the user needs to select which device(s), among different coaxial endovascular devices (e.g. coaxial guidewires, catheter, diverter/stent, sheath etc.), to use and the robot to actuate accordingly.

Many companies are working on endovascular robots that allow users to control and switch between three or more endovascular devices. As part of the endovascular procedure, the user needs to be able to select which device or combination of devices they want to control. Current solutions usually consist of a controller such as a joystick to manipulate the devices' movement and a set of buttons on a control panel surface next to the joystick for selecting which device to control. While this interaction works, it is slow and cumbersome because the user has to let go of the joystick and look down at the control panel to select which device they want to use. Other solutions have multiple joysticks, each mapped to a single device. While this simplifies the selection, it takes up more space and the user has to switch between joysticks. This solution becomes particularly cumbersome if there are four or more devices to control. Furthermore, there is a safety risk associated with the use of multiple joysticks. Specifically, when switching hands between joysticks, unintended joysticks may be accidentally bumped.

It is very important that the user interface (UI) of the robot shows some flexibility in its use to allow a greater ease of use, such as an easy selection / de-selection of the devices during procedure which prevents the user to leave his/her eyes from the screen. Physicians have indeed stated that they do not like to pause and look down to select different devices as it disrupts their flow and focus.
Furthermore, the user interaction should be optimized in terms of speed and efficiency

US 2015/351864 A1 discloses a surgical robot which enables an operator to judge which medical instrument is selected. This surgical robot has a robot body which selectively operates a plurality of medical instruments, an input unit configured to input control information of the robot body, the input unit being common among the plurality of medical instruments, and a display unit configured to display an image of a surgical site of a patient, the display unit having a selecting situation display function which displays a selecting situation of the plurality of medical instruments. The input unit of the surgical robot may comprise a joystick with an operation button provided to an end portion of the joystick, which may be pressed so as to select a desired medical instrument. In particular, US 2015/351862 A1 discloses that once a pointer is moved to the position of a desired selection switch of a slave screen by operating the joystick, the operation button may be pressed so as to select a desired medical instrument.

Hence, the surgical robot disclosed in US 2015/351864 A1 requires a use of the joystick to move a virtual pointer and to push said operation button to select the desired device, i.e. two steps. A simpler selection for using a desired medical device would be desirable for the user.

### SUMMARY OF THE INVENTION

It is an object of the present invention to provide a system for controlling a plurality of (coaxial) endovascular devices, which is easy and quick to use and which does not require the user to take their eyes off the screen or their hands off the joystick.

In a first aspect of the present invention a system for controlling a plurality of (coaxial) endovascular devices is presented, the system comprising:
- a joystick with a plurality of buttons configured to be actuated while the joystick is being handled, and
- a controller configured to associate the plurality of buttons with the plurality of devices and to select and/or deselect one or more of the devices in accordance with the actuation of the buttons for controlling the one or more devices.

A joystick may be any user interfacing device, at least partly manually manipulable, comprising elements to provide signals representative of said manual manipulations and adapted to communicate those signals to a controller in such a way that the controller can directly or indirectly interpret the manipulations and can directly or indirectly trigger action accordingly.

In a second aspect of the present invention a method for controlling a plurality of (coaxial) endovascular devices is presented, the method comprising:
- providing a joystick with a plurality of buttons configured to be actuated while the joystick is being handled,
- associating the plurality of buttons with the plurality of devices, and
- selecting and/or deselecting one or more of the devices in accordance with the actuation of the buttons for controlling the one or more devices.

In yet a further aspect of the present invention, there is provided a corresponding computer program which comprises program code means for causing a computer to perform the steps of the method disclosed herein when said computer program is carried out on a computer as well as a non-transitory computer-readable recording medium that stores therein a computer program product, which, when executed by a processing unit, causes the method disclosed herein to be performed.

Preferred embodiments of the invention are defined in the dependent claims. It shall be understood that the claimed method, computer program and medium have similar and/or identical preferred embodiments as the claimed system, in particular as defined in the dependent claims and as disclosed herein.

The present invention is based on the idea to design a hardware and software new user interface which allows the user controlling to quickly select endovascular devices, switch the devices and steer the devices without needing to take their eyes off the screen or their hands off the joystick. It allows users to do more than what they can currently physically do with two hands (and conventional joysticks). Hence, the present invention allows for more control of endovascular devices.

**In** other words, the invention consists of a hardware element and software element and is about a user interface, UI, system configured to control multiple coaxial (endovascular) devices via at least one joystick arranged to manipulate several devices (e.g. via a motorized (robotic) system adapted to move these devices). The system comprises said at least one joystick provided with a plurality of buttons arranged to be selectable by a finger, especially when the joystick is handled. The joystick may comprise any one of a manual handle, a manually driven element, a thumbstick, a lever, a paddle, a wheel, or any other control interface configured to receive user input. In general, it is not necessary that the joystick is configured to be handled by one or more fingers. It may likewise be handled by other body parts of a user, such as a foot.

For controlling the one or more devices, the devices first have to be selected or deselected in accordance with an actuation of the buttons. However, to be able to perform the selection/deselection it is necessary that the plurality of buttons is associated with the plurality of devices. The association may preferably correspond to a one-to-one mapping or correspondence, meaning that a single button is associated with a single device. That is, actuating a first button may select the first device, actuating a second button may select a second device etc. However, it is also possible for multiple buttons to be associated with a single device, or for a single button to be associated with multiple devices. The association may also be called pairing or linking.

Preferably, the joystick comprises four buttons which mirror four endovascular devices to be controlled. However, another number, for example, eight or even more devices may be controlled by the system presented herein. For example, if there are eight devices to be controlled, the joystick may comprise a double row of four buttons, each button corresponding to one of the devices.

The shape and arrangement of the buttons the joystick is chosen such that it allows the user to select associated device(s) by actuating (e.g. pressing) the buttons without having to look at the buttons.

Preferably, the system presented is paired with (or even comprises) an endovascular robot comprising (modular) robotic arms to manipulate/control the endovascular devices.

The present disclosure is applicable to numerous and various diagnostic, therapeutic and surgical procedures utilizing an endoscope including, but not limited to, anoscopy, arthroscopy, bronchoscopy, colonoscopy, colposcopy, cystoscopy, esophagoscopy, gastroscopy, laparoscopy, laryngoscopy, neuroendoscopy, proctoscopy, sigmoidoscopy and thoracoscopy.

In an embodiment of the system, the buttons are provided on a top surface of the joystick and/or one or more of the plurality of buttons have a different shape and/or color than one or more other buttons of the plurality of buttons. However, the buttons may likewise be provided on a side surface of the joystick or on both top and side surface.

In general, an arrangement of the buttons on the top surface of the joystick is particularly advantageous from an ergonomic perspective, as the buttons are especially easily accessible for the user in this position. Depending on the type of joystick, the same argumentation may apply to the side surface.

Different shapes of the buttons may contribute to better distinguishability of the buttons. Accordingly, the likelihood of confusing different buttons and, consequently, different devices, can be reduced. For example, a second and third button in a row of four buttons may have a slightly different shape, so that it is easier for a user to distinguish said (middle) buttons. The first and fourth button in said row as the outer buttons, can, due to their position, likewise easily felt/recognized.

In a preferred embodiment of the system, the top surface is substantially flat. Hence, the top surface may be absolutely flat or may comprise a slight curvature, particularly a convex curvature.

In another preferred embodiment, the buttons are arranged in a linear-pattern layout or cross-pattern layout on the joystick.

That is, the buttons may be arranged on the joystick in one or more lines. For example, the joystick may comprise four buttons in a (particularly straight) line and possibly four other buttons in another (particularly straight) line, wherein both lines are preferably arranged in a parallel manner. However, joystick may likewise comprise four buttons that are arranged in a cross shape, with two opposing buttons each.

In an embodiment of the system, the system further comprises a display unit configured to provide visual feedback of the selection and/or deselection of the one or more devices.

Thus, the display may particularly show the order / arrangement of buttons on the joystick as well as their associated device(s) and the selection/deselection of said device(s). This way the user can easily recognize which button(s) correspond to which device(s). Furthermore, by displaying the (de)selection, the user can confirm that their device choice is the desired one.

Preferably, the display is further configured to show an image of a body region in which the endovascular devices are located.

In a preferred embodiment of the system, the display unit is further configured to display the layout, shape and/or color of the buttons on the joystick and/or to display an indicator for the buttons of the one or more selected and/or deselected devices.

Hence, the visual feedback of the display may particularly comprise the order/arrangement of buttons on the joystick as well as their associated device(s) and the selection/deselection of said device(s). To this end, for each button there may be displayed a corresponding field, sign or other indicator on the display. If one device is selected, the field on the display corresponding to said device may be highlighted/indicated with a particular color, for example. Selected devices may likewise be highlighted by a frame around their corresponding fields or via blinking. Other indicators are conceivable as well.

Displaying the layout of the buttons and/or their (de)selection may lead to a very intuitive operation of the system for the user.

In another preferred embodiment, the joystick is configured to be moved in a first direction and/or second direction and/or to be rotated around a joystick axis.

In particular, the joystick may be configured to be moved forward and/or backward and/or to be rotated around a joystick axis.

In another embodiment, the controller is configured to control the one or more selected devices to move forward or backward when the joystick is moved forward or backward, and/or wherein the controller is configured to control the one or more selected devices to be rotated when the joystick is rotated.

In general, the controller may be configured to control the one or more selected devices to move in a first direction when the joystick is moved in said first direction, wherein the first direction may generally be any direction. Accordingly, the first direction may not only represent a forward or backward direction, but also a direction to the right or left. In fact, if the joystick was pressed downward or pulled upwards, the controller may be configured to move the selected device(s) downwards or upwards as well.

The speed at which the joystick is moved may also be proportional to the speed of the movement of the selected device(s) the controller controls.

Preferably, the controller is configured to control a selected device /selected devices to rotate around its/their main axis, i.e. to twist, when the joystick is rotated, i.e. twisted, around a joystick axis (e.g. main axis of the joystick). The direction of rotation may be the same for both joystick and device(s) or may be an opposite direction. The function of rotation is also referred to as "rolling".

If two or more devices are selected, the selected devices may be linked together by the controller and when the joystick is pushed forward, for example, the linked devices will move forward together. If the joystick is pulled back, the devices will move back together.

In an embodiment, the controller is configured to select and/or deselect the devices in accordance with an actuation type, wherein the actuation type comprises any one of a strength, duration and/or pattern of the actuation, wherein the actuation pattern comprises an actuation rhythm, an actuation sequence according to a timing pattern and/or a combination of two or more buttons.

For example, a device may only be selected if its corresponding (i.e. associated) button(s) is pressed for more than a predetermined time (e.g. two seconds). Similarly, only if a combination of two or more buttons is actuated at the same time, an associated device may be selected. In another example, when the user twists the joystick, only the device which was first selected in a temporal sequence of selections, will be rotated. The other selected devices will not rotate.

In a preferred embodiment of the system, the controller is configured to associate a first button of the plurality of buttons with the plurality of devices and to select a first device of the plurality of devices and to deselect all other devices of the plurality of devices when the first button is actuated with a first actuation type, and/or the controller is configured to associate a second button of the plurality of buttons with the plurality of devices and to select the plurality of devices when the second button is actuated with a second actuation type.

Additionally or alternatively, the controller may be configured to associate a third button of the plurality of buttons with the plurality of devices and to select the plurality of devices when the third button is actuated with a third actuation type for controlling the plurality of devices to move linearly (e.g. to move forward or backward when the joystick is moved forward or backward) and for further controlling a third device of the plurality of devices to rotate.

In an embodiment, the system further comprises a storage unit configured to store selection information comprising information about the selection and/or deselection of the one or more devices.

In the storage unit there may also be stored controlling information of the selected one or more devices, the controlling information comprising information about movements (comprising linear movements and/or rotations) of the selected devices.

In an embodiment, the controller is configured to restore, using the selection information, a previous selection, selected by actuating a third button of the plurality of buttons, when the third button is actuated for a second time.

In another embodiment, the joystick further comprises a preset button, wherein if the preset button is actuated for a first time the storage unit is configured to store a current selection of the one or more devices and if the preset button is actuated for a second time the controller is configured to select the one or more devices in accordance with the stored selection.

The preset button may be arranged in the vicinity of the other buttons of the device, particularly on a same side of the joystick as the other buttons. Accordingly, the preset button may be arranged on the top surface of the joystick. However, it may likewise be positioned away from the other buttons for better distinguishing the buttons. Similarly, the shape and/or color of the preset button may be the same or different as the shape and/or color of the other buttons. Using the preset button users may create a configuration of device selection they desire and then do a long press, for example, on the preset button. This will save the configuration (in a storage unit, for example). When the user presses the button again the controller may be configured to call up the preset configuration, i.e. to select the devices according to the stored configuration.

In an embodiment of the method, the method further comprises controlling the one or more selected devices to move forward or backward when the joystick is moved forward or backward, and/or wherein the controller is configured to control the one or more selected devices to be rotated when the joystick is rotated.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a system according to the present invention;
Fig. 2 shows a schematic diagram of a second embodiment of a system according to the present invention;
Fig. 3A shows the joystick of the second embodiment of the system shown in Fig. 2 is a first perspective view;
Fig. 3B shows the joystick of the second embodiment of the system shown in Fig. 2 in a second perspective view;
Fig. 4 shows an embodiment of a display of a third embodiment of a system according to the present invention;
Fig. 5 shows an embodiment of a joystick 12 of a fourth embodiment of a system according to the present invention; and
Fig. 6 shows a flow chart of an embodiment of a method according to the present invention.

### DETAILED DESCRIPTION OF EMBODIMENTS

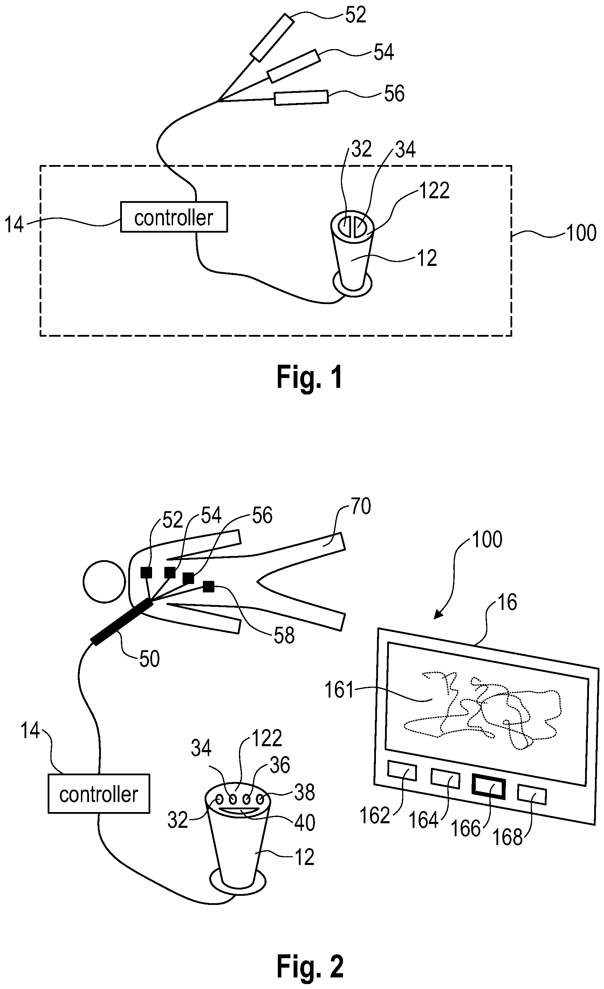

Fig. 1 shows a schematic diagram of a first embodiment of a system 100 according to the present invention. The system 100 comprises a joystick 12 and a controller 14.

The joystick 12 has a shape which is designed similarly to a car's gearstick. It is hardware that is arranged on a desktop. However, the joystick may likewise be clipped to the side of an iGT (interventional Guided Therapy) table. The joystick 12 comprises two buttons 32 and 34 on its top surface 122 which are configured to be actuated, particularly pressed, by a user. The controller 14 of the system 100 is connected to the joystick via a cable connection 13. However, the joystick 12 and the controller may be connected wirelessly as well.

Via the cable connection 13 (or in a wireless manner) button data and actuation data of the joystick 12 may the provided to the controller 14 for further processing. The button data may comprise information about the buttons 32 and 34 of the joystick 12, particularly information about existence of said buttons 32 and 34. The actuation data may comprise information about the actuation of the buttons 32 und 34, particularly including information about which of the buttons has been / is actuated/pressed. Actuation data may likewise comprise information about an actuation type, wherein the information about the actuation type comprises information on any one of a strength, duration and/or pattern of the actuation (of the buttons 32 und 34), wherein the actuation pattern comprises an actuation rhythm, an actuation sequence according to a timing pattern and/or a combination of the buttons 32 and 34.

The controller 14 is configured to obtain (retrieve or receive) the buttons data and actuation data from the joystick 12. To this end, the controller 14 may comprise e.g. a (wired or wireless) communication interface or data interface, such as a Bluetooth interface, Wi-Fi interface, LAN interface, HDMI interface, direct cable connection (this is shown in Fig. 1), or any other suitable interface allowing data transfer to the controller 14. The controller 14 may associate the two of buttons 32 und 34 with the endovascular devices 52 and 54 (based on the button data) and to select and/or deselect (based on the actuation data) one or both of the devices 52 and 54 in accordance with the actuation of the buttons 32 and 34 for controlling the devices 52 and 54. The controller 14 may comprise circuitry, e.g. a processor, processing circuitry, a computer, dedicated hardware, etc. that carries out the functions of the controller 14. Separate units or elements may be used that together represent circuitry of the controller 14.

For example, the controller 14 may be configured to associate the first button 32 with the first device 52 and the second button with the second device 54. If the first button is pressed the first device 52 may be selected by the controller 14 and if the second 34 button is pressed the second device 54 may be selected by the controller 14. Furthermore, if both buttons 32 and 34 are pressed simultaneously both devices 52 and 54 may be selected. If the first device 52 is selected and the joystick 12 is pushed forward the first device may be pushed forward by the controller 14. Similarly, if the first device 52 is selected and the joystick 12 is pulled backward the first device 52 may be pulled backward. The same may apply to the second device 54 and to other movement directions or even other types of movements, such as a rotation.

In general, for controlling the devices 52 and 54, the controller 14 may be connected to said devices via a wired or wireless communication interface or data interface, such as a Bluetooth interface, Wi-Fi interface, LAN interface, HDMI interface, direct cable connection (this is shown in Fig. 1), or any other suitable interface allowing data transfer.

Fig. 2 shows a schematic diagram of a second embodiment of a system 100 according to the present invention. In this embodiment, the system 100 comprises a joystick 12, a controller 14 and a display unit 16. The system 100 may further comprise an endovascular robot 50 whose arms comprise a first endovascular device 52, a second endovascular device 54, a third endovascular device 56 and a fourth endovascular device 58, respectively.

The joystick 12 comprises four buttons 32, 34, 36 and 38 on its top surface 122 which are configured to be actuated by a user. Based on data (particularly button data and actuation data as described above) obtained from the joystick 12, the controller 14 is configured to select and/or deselect one or more of the four endovascular devices 52, 54, 56 and 58. Once selected by the controller the endovascular devices 52, 54, 56 and 58 attached to the arms of the endovascular robot 50 may be controlled by the controller, particularly be moved (inside the patient 70) by the controller 12 in accordance with a movement of the joystick 12.

On the display unit 16 there may be shown a first field 161 comprising an image of an of a body region inside the patient where the endovascular devices 52, 54, 56 and 58 may be located. Furthermore, the display unit 16 is configured to display indicators 162, 164, 166 and 168 for the buttons 32, 34, 36 and 38, wherein the first indicator 162 corresponds to the first button 32, the second indicator 164 corresponds to the second button 34 and so on. The layout of the buttons 32, 34, 36 and 38 on the joystick 12 corresponds to a line on which the buttons are lined up. On the display unit 16 the layout of the buttons 32, 34, 36 and 38 is reflected by their corresponding indictors, which are shown in a line below the body region displayed. As can be seen, the third indicator 166 comprises a bold frame, whereas the frame of the other indicators 162, 164 and 168 is a given by a thin line. In this embodiment, this indicates that the button corresponding to the third indicator 166, i.e. the third button 36, is / has been actuated. The controller 14 associates the first button 32 with the first endovascular device 52, the second button 34 with the second endovascular device 54, the third button 36 with the third endovascular device 56 and the fourth button 38 with the fourth endovascular devices. By the bold frame of the third indicator 166 it is also indicated that the third endovascular device 56 is selected. The selection of the third endovascular device 56 may also be indicated in another manner, for example, by displaying the indicator 166 in another color than the other indicators.

Apart from the four buttons 32, 34, 36 and 38 the joystick 12 further comprises a preset button 40 on its top surface 122. In case the preset button 40 is actuated for a first time the storage unit is configured to store a current selection of the devices 32, 34, 36 and 38 and if the preset button is actuated for another time the controller 14 is configured to restore the stored device selection. The preset button 40 allows a user to quickly access a pre-configured selection and essentially is a shortcut to save time and improve efficiency by eliminating repetitive manual selections.

In general, selection and control of the devices 52, 54, 56 and 58 may be based on an actuation type of the buttons 32, 34, 36 and 38. For example, a quick selection of one of the devices may be achieved by double clicking. If several devices are selected and users would like to deselect everything and only select a single device, they can double click on the button corresponding to the desired device and the system will deselect all other devices and only select the device which the user has double clicked on. For returning to a previous selection, after the user has double clicked to select the desired device, the user may double click again on the same button (corresponding to the desired device) to return to the previous selection of devices, i.e. to the selection prior to the first double click. For selecting all devices (at the same time), the user may press anyone of the plurality of buttons on the top surface of the joystick 12 with a long press. The controller 14 may then particularly control the device corresponding to the long press. To be more precise, when all devices are selected, all devices may be controlled to translate (move forward and/or backward together), for example, but only one device, the one device "selected" via long-press, may rotate, for example. Hence, by long press on the button corresponding to the device to be rotated, all devices may be selected to obtain a first control signal (for moving linearly) and one of the plurality of the devices (the device corresponding to the button that has been long-pressed) may obtain a second control signal (for rotating). In other words, if a button corresponding to "device 2" is pressed by a user for long, all devices will be selected and controlled to move forward and/or backward together, but only "device 2" will be controlled to rotate and the other devices may remain stationary. Of course, the "long press" may be replaced by another actuation type.

Hence, the selection/deselection of the endovascular devices may not only based on whether buttons are actuated but also on the actuation type with which the buttons are actuated. In other words, the "type" of actuations/clicks are representative of a type of "shortcut" the user is intending to perform. Therefore, a single click, a double click, a triple click, long press etc. on a single button or quick sequential pressing of several buttons may lead to different (de)selections of the buttons.

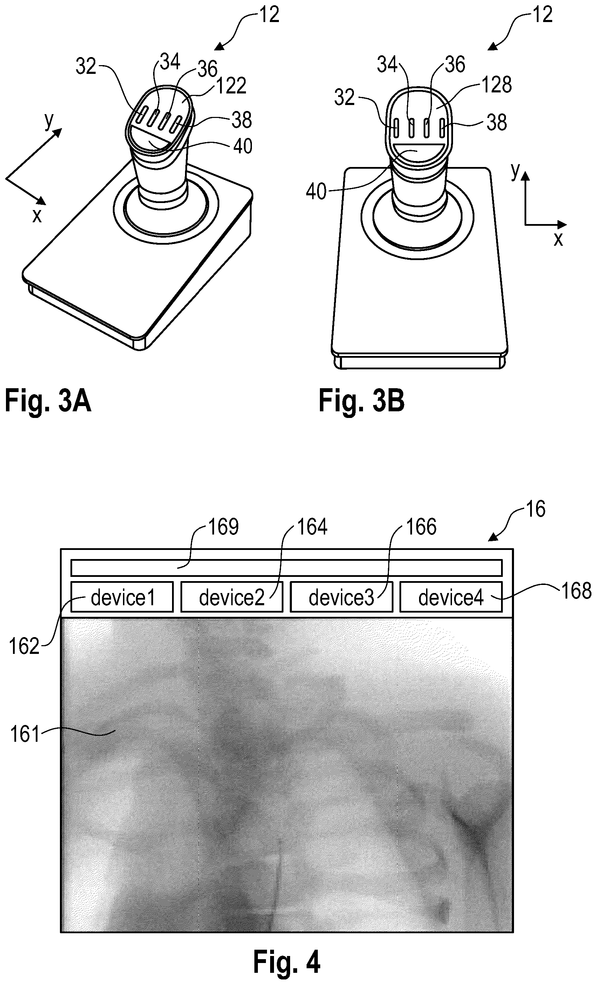

Fig. 3A shows the joystick 12 of the second embodiment of the system 100 shown in Fig. 2 is a first perspective view. The joystick 12 comprises articulation in the y-axis and at least the top part of the joystick 12 can be rotated +/- 15°. Movements of the joystick 12 in y-direction or as rotation is linked to the movement of the device(s) selected via the (push) buttons 32, 34, 36 and 38 on the top surface 122 of the joystick 12. Software of the controller 14 (shown in Fig. 2) is configured to detect the movement of the joystick 12 and the button(s) pressed and translates it as command for the endovascular robot 50 / the devices 52, 54, 56 and 58.

Fig. 3B shows the joystick 12 of the second embodiment of the system 100 shown in Fig. 2 in a second perspective view.

Fig. 4 shows an embodiment of a display 16 of a third embodiment of a system 100 according to the present invention. The display unit 16 may e.g. be a monitor or screen of a PC, workstation, laptop tablet, etc. In a first field 161 the display 16 shows an image of a body region inside a patient in which four coaxial endovascular devices are currently located. Above the field 161 indicators 162, 164, 166 and 168 for each of said four devices are displayed as four different fields. More specifically, in Fig. 4 the display unit 16 shows an x-ray image in the filed 161 and above the x-ray image there are elements showing the name of the different devices, preferably laid out in the same pattern as the joystick buttons. This makes it easy for user to look at the display unit 16 and see which endovascular device they would like to select, press joystick button to select and look at the screen to confirm their selection. Above the four indicators 162, 164, 166 and 168 there is furthermore displayed another field 169 which is configured to display patient information, for example. A coloring or blinking behavior of the indicators 162, 164, 166 and 168 may indicate which corresponding endovascular device(s) are currently selected.

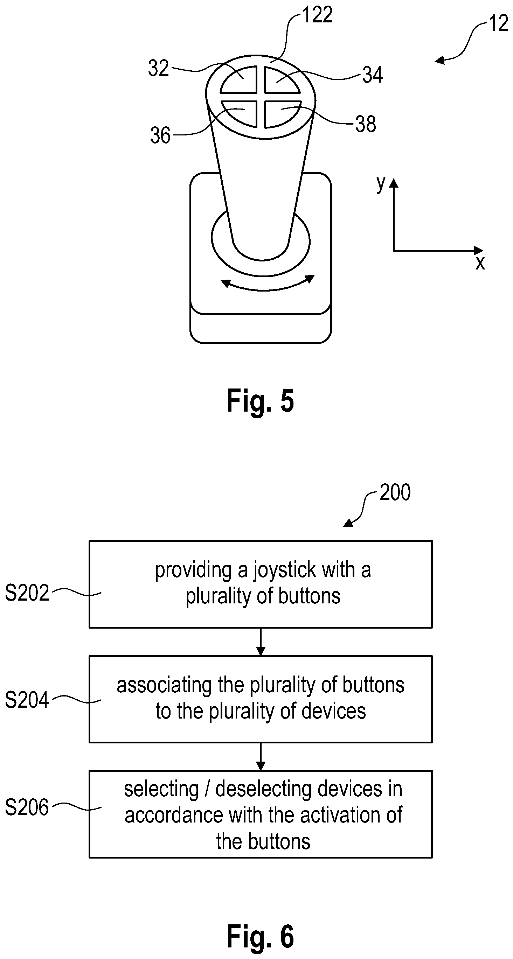

Fig. 5 shows an embodiment of a joystick 12 of a fourth embodiment of a system 100 according to the present invention. In this embodiment, rather than a linear configuration of buttons, the four buttons 32, 34, 36 and 38 are arranged in a cross pattern on the top surface 122 of the joystick (and the indicators on a display 16 of the system 100 may be arranged accordingly). This arrangement/layout of the buttons 32, 34, 36 and 38 also allows for an easy and quick selection and switching. In this embodiment, the joystick may be moved forward or backward (y-direction) and/or to the left and right (x-direction). Furthermore, as indicated by the double arrow, the joystick may be twisted/rotated clockwise or counterclockwise (leading to the selected device(s) being rotated in the same direction, for example).

Fig. 6 shows a flow chart of an embodiment of a method 200 according to the present invention.

In a first step S202 of the method a joystick with a plurality of buttons configured to be actuated while the joystick is being handled is provided.

In a second step S204 of the method the plurality of buttons is associated with the plurality of devices. The association may be a one-to-one association or a many-to-one-association, i.e. a single button may be associated with two or more devices or vice versa.

In a third step S206 one or more of the devices are selected and/or deselected in accordance with the actuation of the buttons for controlling the one or more devices.

In general, depending on the type of the actuation of the buttons not only the selection/deselection of buttons may be changed, but also the association of the buttons to the devices.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. System for controlling a plurality of endovascular devices, comprising:
- a joystick with a plurality of buttons configured to be actuated while the joystick is being handled, and
- a controller configured to associate the plurality of buttons with the plurality of devices and to select and/or deselect one or more of the devices in accordance with the actuation of the buttons for controlling the one or more devices.

2. System according to claim 1, wherein the buttons are provided on a top surface of the joystick and/or wherein one or more of the plurality of buttons have a different shape and/or color than one or more other buttons of the plurality of buttons.

3. System according to claim 2, wherein the top surface is substantially flat.

4. System according to any one of the preceding claims, wherein the buttons are arranged in a linear-pattern layout or cross-pattern layout on the joystick.

5. System according to any one of the preceding claims further comprising
- a display unit configured to provide visual feedback of the selection and/or deselection of the one or more devices.

6. System according to claim 5, wherein the display unit is further configured to display the layout, shape and/or color of the buttons on the joystick and/or to display an indicator for the buttons of the one or more selected and/or deselected devices.

7. System according to any one of the preceding claims, wherein the joystick is configured to be moved in a first direction and/or second direction and/or to be rotated around a joystick axis.

8. System according to claim 7, wherein the controller is configured to control the one or more selected devices to move forward or backward when the joystick is moved forward or backward, and/or wherein the controller is configured to control the one or more selected devices to be rotated when the joystick is rotated.

9. System according to any one of the preceding claims, wherein the controller is configured to select and/or deselect the devices in accordance with an actuation type, wherein the actuation type comprises any one of a strength, duration and/or pattern of the actuation, wherein the actuation pattern comprises an actuation rhythm, an actuation sequence according to a timing pattern and/or a combination of two or more buttons.

10. System according to claim 9, wherein the controller is configured to associate a first button of the plurality of buttons with the plurality of devices and to select a first device of the plurality of devices and to deselect all other devices of the plurality of devices when the first button is actuated with a first actuation type, and/or wherein the controller is configured to associate a second button of the plurality of buttons with the plurality of devices and to select the plurality of devices when the second button is actuated with a second actuation type.

11. System according to any one of the preceding claims, further comprising a storage unit configured to store selection information comprising information about the selection and/or deselection of the one or more devices.

12. System according to claim 11, wherein the controller is configured to restore, using the selection information, a previous selection, selected by actuating a third button of the plurality of buttons, when the third button is actuated for a second time.

13. System according to claim 11 or 12, wherein the joystick further comprises a preset button, wherein if the preset button is actuated for a first time the storage unit is configured to store a current selection of the one or more devices and if the preset button is actuated for a second time the controller is configured to select the one or more devices in accordance with the stored selection.

14. Method for controlling a plurality of endovascular devices, comprising:
- providing a joystick with a plurality of buttons configured to be actuated while the joystick is being handled,
- associating the plurality of buttons with the plurality of devices, and
- selecting and/or deselecting one or more of the devices in accordance with the actuation of the buttons for controlling the one or more devices.

15. Method according to claim 14, further comprising controlling the one or more selected devices to move forward or backward when the joystick is moved forward or backward, and/or wherein the controller is configured to control the one or more selected devices to be rotated when the joystick is rotated.
